# EUROPEAN PATENT SPECIFICATION

(11) **EP 0 733 030 B1**
(45) Date of publication and mention of the grant of the patent: **31.10.2001**
(21) Application number: 94924461.0
(22) Date of filing: 21.07.1994
(51) Int. Cl.: C07C 5/05, C07C 5/08, C07C 5/09

(54) **SELECTIVE HYDROGENATION OF HIGHLY UNSATURATED COMPOUNDS IN HYDROCARBON STREAMS**
SELEKTIVE HYDRIERUNG VON HOCH UNGESÄTTIGTEN VERBINDUNGEN IN KOHLENWASSERSTOFFSTRÖMEN
HYDROGENATION SELECTIVE DE COMPOSES HAUTEMENT INSATURES DANS UN COURANT D'HYDROCARBURES

(30) Priority: 08.12.1993 US 163311
(43) Date of publication of application: 25.09.1996
(73) Proprietor: CHEMICAL RESEARCH & LICENSING COMPANY, Pasadena, Texas 77507 (US)
(72) Inventor: HEARN, Dennis, Pasadena, TX 77507 (US); ARGANBRIGHT, Robert, P., Pasadena, TX 77507 (US); JONES, Edward, M., Jr., Pasadena, TX 77507 (US); SMITH, Lawrence, A., Jr., Pasadena, TX 77507 (US); GILDERT, Gary, R., Pasadena, TX 77507 (US)
(74) Representative: Zumstein, Fritz, Dr.
(86) International application number: US9407758
(87) International publication number: WO9515934

(56) References cited:
- EP-A- 0 556 025
- GB-A- 835 689
- US-A- 4 215 011
- US-A- 4 221 653
- US-A- 4 232 177
- US-A- 4 439 350
- US-A- 5 087 780

## Description

The present invention relates to the selective hydrogenation of diolefins and acetylenic compounds in a olefin rich stream. More particularly the invention relates to a process utilizing a hydrogenation catalyst in a structure to serve as both the catalyst and as a distillation structure for the simultaneous reaction and separation of the reactants and reaction products.

### Related Art

Mixed refinery streams often contain a broad spectrum of olefinic compounds. This is especially true of products from either catalytic cracking or thermal cracking processes. These unsaturated compounds comprise ethylene, acetylene, propylene, propadiene, methyl acetylene, butenes, butadiene, amylenes, hexenes etc. Many of these compounds are valuable, especially as feed stocks for chemical products. Ethylene, especially is recovered. Additionally, propylene and the butenes are valuable. However, the olefins having more than one double bond and the acetylenic compounds (having a triple bond) have lesser uses and are detrimental to many of the chemical process in which the single double bond compounds are used, for example polymerization. Over the range of hydrocarbons under consideration, the removal of highly unsaturated compounds is of value as a feed pretreatment, since these compounds have frequently been found to be detrimental in most processing, storage and use of the streams.

The C₄ cuts are sources of alkanes and alkenes for paraffin alkylation to produce C₈ gasoline blending components and as feeds for ether production.

The C₅ refinery cut is valuable as a gasoline blending stock or as source of isoamylene to form an ether by reaction with lower alcohols. Tertiary amyl methyl ether (TAME) is rapidly becoming valuable to refiners as a result of the recently passed Clean Air Act which sets some new limits on gasoline composition. Some of these requirements are (1) to include a certain amount of "oxygenates", such as methyl tertiary butyl ether (MTBE), TAME or ethanol, (2) to reduce the amount of olefins in gasoline, and (3) to reduce the vapor pressure (volatility).

The C₅'s in the feed to a TAME unit are contained in a single "light naphtha" cut which contains everything from C₅'s through C₈'s and higher. This mixture can easily contain 150 to 200 components and thus identification and separation of the products is difficult. Several of the minor components (diolefins) in the feed will react slowly with oxygen during storage to produce "gum" and other undesirable materials. However, these components also react very rapidly in the TAME process to form a yellow, foul smelling gummy material. Thus it is seen to be desirable to remove these components whether the "light naphtha" cut is to be used only for gasoline blending by itself or as feed to a TAME process.

The use of a solid particulate catalyst as part of a distillation structure in a combination distillation column reactor for various reactions is described in U.S. Pat. No.s: (etherification) 4,232,177; 4,307,254; 4,336,407; 4,504,687; 4,918,243; and 4,978,807; (dimerization); 4,242,530; (hydration) 4,982,022; (dissociation) 4,447,668; and (aromatic alkylation) 4,950,834 and 5,019,669. Additionally U.S. Pat. No.s 4,302,356 and 4,443,559 disclose catalyst structures which are useful as distillation structures.

Hydrogenation is the reaction of hydrogen with a carbon-carbon multiple bond to "saturate" the compound. This reaction has long been known and is usually done at super atmospheric pressures and moderate temperatures using a large excess of hydrogen over a metal catalyst. Among the metals known to catalyze the hydrogenation reaction are platinum, rhenium, cobalt, molybdenum, nickel, tungsten and palladium. Generally, commercial forms of catalyst use supported oxides of these metals. The oxide is reduced to the active form either prior to use with a reducing agent or during use by the hydrogen in the feed. These metals also catalyze other reactions, most notably dehydrogenation at elevated temperatures. Additionally they can promote the reaction of olefinic compounds with themselves or other olefins to produce dimers or oligomers as residence time is increased.

Selective hydrogenation of hydrocarbon compounds has been known for quite some time. Peterson, et al in "The Selective Hydrogenation of Pyrolysis Gasoline" presented to the Petroleum Division of the American Chemical Society in September of 1962, discusses the selective hydrogenation of C₄ and higher diolefins. Boitiaux, et al in "Newest Hydrogenation Catalyst", Hydrocarbon Processing, March 1985, presents a general, non enabling overview of various uses of hydrogenation catalysts, including selective hydrogenation of a propylene rich stream and other cuts. Conventional liquid phase hydrogenations as presently practiced required high hydrogen partial pressures, usually in excess of 1378 KPa (200 psi) and more frequently in a range of up to 2756 KPa (400 psi) or more. In a liquid phase hydrogenation the hydrogen partial pressure is essentially the system pressure.

U.S. Pat. No. 2,717,202 to Bailey discloses a countercurrent process for the hydrogenation of lard carried out in a plurality of independent vertical chamber using a pumped catalyst under undisclosed pressure conditions. U.S. Pat. No. 4,221,653 to Chervenak et al discloses a concurrent hydrogenation for using an ebullating bed at extremely high pressures. UK Patent Specification 835,689 discloses a high pressure, concurrent trickle bed hydrogenation of C₂ and C₃ fractions to remove acetylenes.

U.S. Pat No. 5,087,780 to Arganbright disclosed a process for the hydroisomerization of butenes using an alumina supported palladium oxide catalyst arranged in a structure for use as both the catalyst and distillation in a catalytic distillation reactor. The hydrogenation of dienes was also observed under high hydrogen partial pressure, in excess of (70 psia) 482.3 KPa, but not at around (10 psia) 68.9 KPa. EP-A-556 025 describes the selective hydrogenation of diolefins in a light naphtha at certain conditions of system pressure and temperature, however does not disclose the hydrogen partial pressure.

It is an advantage of the present process that the diolefins (dienes) and acetylenic compounds contained within the hydrocarbon stream contacted with the catalyst are converted to olefins or alkanes with very little if any formation of oligomers or little if any saturation of the mono-olefins.

### SUMMARY OF THE INVENTION

The object of the present invention is a process for the selective hydrogenation of highly unsaturated compounds comprising feeding a hydrocarbon stream containing monoolefins together with highly unsaturated compounds which comprise diolefins and acetylenes along with a hydrogen stream at an effectuating hydrogen partial pressure in the range of least (0.1 psia) 0.69 KPa to less than (10 psia) 68.9 KPa to a distillation column reactor containing a hydrogenation catalyst which is a component of a distillation structure, selectively hydrogenating a portion of the highly unsaturated compounds and operating at an internal reflux in the range of 0.2 to 20 L/D.

Within the hydrogen partial pressures as defined no more hydrogen than necessary to maintain the catalyst (most likely to reduce the catalyst metal oxide and maintain it in the hydride state) and hydrogenate the highly unsaturated compounds is employed, since the excess hydrogen is usually vented. This preferably is a hydrogen partial pressure in the range of about (0.1 to 7 psia) 0.689 to 48.23 KPa. Optimal results have been obtained in the range between 104.75 and 135.77 KPa (0.5 and 5 psig)hydrogen partial pressure.

The hydrocarbon stream typically comprises C₂ to C₉ aliphatic compounds, which may be narrow cuts or include a range of carbon content. The invention is the discovery that a hydrogenation carried out in a catalytic distillation column requires only a fraction of the hydrogen partial pressure required in the liquid phase processes which are the form of prior commercial operation for this type of stream, but give the same or better result. Thus the capital investment and operating expense for the present hydrogenation are substantially lower than prior commercial operations.

Without limiting the scope of the invention it is proposed that the mechanism that produces the effectiveness of the present process is the condensation of a portion of the vapors in the reaction system, which occludes sufficient hydrogen in the condensed liquid to obtain the requisite intimate contact between the hydrogen and the highly unsaturated compounds in the presence of the catalyst to result in their hydrogenation.

The highly unsaturated compounds may be present in very minor amounts, i.e., a few parts per million up to major amounts, i.e., over 90 weight %. The present invention may be used to remove impurities or to convert commodity amounts of the highly unsaturated compounds into monoolefins or alkanes as desired.

The hydrogen rate must be adjusted at the partial pressure described such that it is sufficient to support the hydrogenation reaction and replace hydrogen lost from the catalyst but kept below that producing hydrogenation of monoolefins which is understood to be the "effectuating hydrogen partial pressure" as that term is used herein.

As can be readily appreciated the amount of the highly unsaturated compound in the hydrocarbon stream is a factor to be considered in selecting the optimum hydrogen partial pressure, since at least a stoichiometric amount of hydrogen must be present in the system to be available for the reaction. When the highly unsaturated compounds are impurities, present in parts per million the lower range of hydrogen partial pressure is a an excess, but it is necessary because of the scarcity of the selective reactant. Also the nature of this reaction between a gas and a liquid and the apparent need to occlude the hydrogen into the liquid makes an excess of hydrogen within the partial pressures a preferred mode of operation.

An additional feature of the process is that a portion of the mono-olefins contained within the stream or produced by the selective hydrogenation of the diolefins may be isomerized to more desirable products. Isomerization can be achieved with the same family of catalysts as used in hydrogenations. Generally the relative rates of reaction for various compounds are in the order of from faster to slower:
(1) hydrogenation of diolefins
(2) isomerization of the mono-olefins
(3) hydrogenation of the mono-olefins.
It has been shown generally that in a stream containing diolefins, the diolefins will be hydrogenated before isomerization occurs. It has also been found that very low total pressures may be used for optimal results in some of the present hydrogenations, preferably in the range of 445.83 to 1134.83 KPa (50 to 150 psig) with the same excellent results. Both higher and lower pressures within the broad range may be used may be used with satisfactory results.

### DETAILED DESCRIPTION AND PREFERRED EMBODIMENTS

Although the hydrogenation reactions have been described as reversible at elevated temperatures above 482°C (900°F) (See for example the Peterson article cited above) under the temperature conditions employed in the present invention, the hydrogenation is not reversible. In the usual application of a process where the catalyst serves as a distillation component, the equilibrium is constantly disturbed, thus driving the reaction toward completion, that is, the reaction has an increased driving force because the reaction products have been removed and cannot contribute to a reverse reaction (LeChatelier's Principle). In the present process where there is no reversible reaction, no benefit is to be derived by removing the products of the reaction to increase the driving force of the reaction by. Similarly the poor performance of prior vapor phase hydrogenations would not suggest the use of distillation type reaction. Thus, it is unexpected that catalytic distillation would be of benefit for non reversible hydrogenation.

It is believed that in the present reaction catalytic distillation is a benefit first, because the reaction is occurring concurrently with distillation, the initial reaction products and other stream components are removed from the reaction zone as quickly as possible reducing the likelihood of side reactions. Second, because all the components are boiling the temperature of reaction is controlled by the boiling point of the mixture at the system pressure. The heat of reaction simply creates more boil up, but no increase in temperature at a given pressure. As a result, a great deal of control over the rate of reaction and distribution of products can be achieved by regulating the system pressure. Also, adjusting the throughput (residence time = liquid hourly space velocity⁻¹) gives further control of product distribution and to a degree control of the side reactions such as oligomerization. A further benefit that this reaction may gain from catalytic distillation is the washing effect that the internal reflux provides to the catalyst thereby reducing polymer build up and coking. Internal reflux may vary over the range of 0.2 to 20 L/D (wt. liquid just below the catalyst bed/wt. distillate) give excellent results, and for the C₃-C₅ streams usually in the range of 0.5 to 4 L/D.

Quite surprisingly the low hydrogen partial pressure used in the distillation system did not result in the failure of the hydrogenation which would have been expected based on the high hydrogen partial pressure found in the liquid phase systems which are the world wide standard. As observed earlier the phenomenon of condensation which is a constant factor in a distillation is believed to result in the same or better hydrogen availability, as the high pressure in the liquid phase, that is, the hydrogen is introduced into the liquid so that the hydrogenation occurs.

In one embodiment the present invention comprises the selective hydrogenation of acetylenic compounds and diolefins contained within a propylene rich stream to purify the stream and obtain greater amounts of the propylene. The propylene rich stream is fed to a distillation column reactor into a reaction distillation zone containing a supported palladium oxide catalyst in the form of a catalytic distillation structure. Hydrogen is provided as necessary to support the reaction and, it is believed, to reduce the oxide and maintain it in the hydride state. Previously the hydride state was believed to be the active state, however, the very low amounts of hydrogen present that give excellent results, may indicate otherwise. In any event the state of the catalyst is a matter of theory relating to mechanism, which is not the subject of the present invention. The distillation column reactor is operated at a pressure such that the reaction mixture is boiling in the bed of catalyst. If desired, a bottoms stream containing any higher boiling material may be withdrawn to effectuate a complete separation.

In a C₃ embodiment, using the hydrogen partial pressure as recited, the present invention includes a process for the selective hydrogenation of the diolefins and acetylenic compounds contained within a propylene rich stream, comprising the steps of:
(a) feeding (1) a first stream comprising propylene, di-olefins and acetylenic compounds and (2) a second stream containing hydrogen to a distillation column reactor into a feed zone;
(b) concurrently in said distillation column reactor
   (i) contacting said first and second streams in a distillation reaction zone with a hydrogenation catalyst capable of acting as a distillation structure thereby reacting essentially all of said diolefins and acetylenic compounds with said hydrogen to form propylene and other hydrogenated products in a reaction mixture, and
   (ii) separating the propylene contained in said first stream and the propylene formed by the reaction of said diolefins and said acetylenic compounds from said reaction mixture by fractional distillation and
(c) withdrawing the separated propylene from step (b)
   (ii) along with any propane and lighter compounds, including any unreacted hydrogen, from said distillation column reactor as overheads. Optionally the process may include withdrawing any C₄ or higher boiling compounds from said distillation column reactor as bottoms. There is no significant loss of propylene from the hydrogenation.

The present invention carries out the method in a catalyst packed column which can be appreciated to contain a vapor phase and some liquid phase as in any distillation. The distillation column reactor is operated at a pressure such that the reaction mixture is boiling in the bed of catalyst. The present process operates at overhead pressure of said distillation column reactor in the range between 0 and 2512.83 KPa (0 and 350 psig), preferably 1823.83 KPa (250) or less and temperatures within said distillation reaction zone in the range of 4.44 to 149°C (40 to 300°F), preferably 43 to 132°C (110 to 270°F) at the requisite hydrogen partial pressures. The feed weight hourly space velocity (WHSV), which is herein understood to mean the unit weight of feed per hour entering the reaction distillation column per unit weight of catalyst in the catalytic distillation structures, may vary over a very wide range within the other condition perimeters, e.g. 0.1 to 35.

The advantages of utilizing a distillation column reactor in the instant selective hydrogenation process lie in the better selectivity of diolefin to olefin, conservation of heat and the separation by distillation which can remove some undesirable compound, e.g. heavy sulfur contaminants, from the feed prior to exposure to the catalyst and the distillation can concentrate desired components in the catalyst zone.

A "froth level" is preferably maintained throughout the catalyst bed by control of the bottoms and/or overheads withdrawal rate which improves the effectiveness of the catalyst thereby decreasing the height of catalyst needed. As may be appreciated the liquid is boiling and the physical state is actually a froth having a higher density than would be normal in a packed distillation column but less than the liquid without the boiling vapors, as described in U.S. Pat. No. 5,221,441.

Basically the froth mode called "liquid phase continuous (LPC)" hereafter is understood to mean that the flow of liquid from the catalytic distillation section has been restricted so that the rising vapor creates a froth. In effect the continuous phase is the liquid rather than the vapor as is usual in a distillation. The result is increased liquid contact with the catalytic material during the distillation and improved selective hydrogenation.

The temperature in the reactor is determined by the boiling point of the liquid mixture present at any given pressure. The temperature in the lower portions of the column will reflect the constitution of the material in that part of the column, which will be higher than the overhead; that is, at constant pressure a change in the temperature of the system indicates a change in the composition in the column. To change the temperature the pressure is changed. Temperature control in the reaction zone is thus effected by a change in pressure; by increasing the pressure, the temperature in the system is increased, and vice versa.

As described the catalytic material employed in the hydrogenation process is in a form to serve as distillation packing. Broadly stated, the catalytic material is a component of a distillation system functioning as both a catalyst and distillation packing, i.e., a packing for a distillation column having both a distillation function and a catalytic function.

The reaction system can be described as heterogenous since the catalyst remains a distinct entity. Any suitable hydrogenation catalyst may be used, for example Group VIII metals of the Periodic Table of Elements as the principal catalytic component, alone or with promoters and modifiers such as palladium/gold, palladium/silver, cobalt/zirconium, nickel preferably deposited on a support such as alumina, fire brick, pumice, carbon, silica, resin or the like.

A preferred catalytic material comprises palladium oxide, preferably 0.1 to 5.0 weight %, supported on an appropriate support medium such as alumina, carbon or silica, e.g., 0.3 cm (1/8") alumina extrudates. In a preferred catalytic distillation structure the particulate catalyst material is disposed within a porous plate or screen to contain the catalyst and provide a distillation surfaces, in the form of a wire mesh structure, such as a wire mesh tubular structure or any other similar structure.

A preferred catalyst structure for the present hydrogenation reaction comprising flexible, semi-rigid open mesh tubular material, such as stainless steel wire mesh, filed with a particulate catalytic material in one of several embodiments recently developed in conjunction with the present process.

One new catalyst structure developed for use in hydrogenations is described in US Pat. No. 5,266,546.

Briefly the new catalyst structure is a catalytic distillation structure comprising flexible, semi-rigid open mesh tubular material, such as stainless steel wire mesh, filed with a particulate catalytic material said tubular material having two ends and having a length in the range of from about one-half to twice the diameter of said tubular material, a first end being sealed together along a first axis to form a first seam and a second end being sealed together along a second axis to form a second seam wherein the plane of the first seam along the axis of said tubular material and the plane of the second seam along the axis of said tubular material bisect each other at an angle of about 15 to 90°.

US Patent No. 4,242,530 and US Pat. No. 4,443,559 disclose supported catalyst in a plurality of pockets in a cloth belt or wire mesh tubular structures, which is supported in the distillation column reactor by open mesh knitted stainless steel wire by twisting the two together into a helix, which have been used. US Patent No. 5,348,710 filed 06/11/93, describes several other suitable structures in the prior art and disclosed new structures suitable for this process.

The particulate catalyst material may be a powder, small irregular chunks or fragments, small beads and the like. The particular form of the catalytic material in the structure is not critical, so long as sufficient surface area is provided to allow a reasonable reaction rate. The sizing of catalyst particles can be best determined for each catalytic material (since the porosity or available internal surface area will vary for different material and of course affect the activity of the catalytic material).

For the present hydrogenations the preferred catalysts structures for the packing are those employing the more open structure of permeable plates or screen wire.

### Example 1

### C₄ STREAMS

This set of runs demonstrates the unexpected diene removal from C₄ streams at extremely low hydrogen partial pressures. It was also demonstrated that lower total pressures were also suitable. The runs were conducted in two modes. In one mode, conventional distillation, a vapor continuous phase was used. In the other mode, a preferred liquid continuous phase "LPC" mode was used.

The reactor used for Run 1 was a 7.62 cm (three inch) diameter column containing 609.6 cm (20 feet) of catalyst packing, containing 0.028 m³ (1 cubic foot) of catalytic material (0.5% pd on 2.38mm - 1.68mm Sieve opening (8-12 mesh) alumina-E144SDU product of Calcicat, Catalyst and Performance Chemicals Division, Mallinckrodt, Inc.), with 137.16 cm (4.5 feet) of 1.59cm (5/8") steel pall rings above and 472.44 cm (15.5 feet) of 1.59cm (5/8") steel Pall rings below the catalyst bed. Run 2 used a 7.62cm (three inch) diameter column containing 609.6cm (20 feet) of catalyst packing, containing 0.028 m³ (1 cubic foot) of catalytic material (0.5% pd on 2.38mm-1.68mm Sieve opening (8-12 mesh) alumina-E144SDU product of Calcicat, Catalyst and Performance Chemicals Division, Mallinckrodt, Inc.), with 137.16 cm (4.5 feet) of 1.59 cm (5/8") steel pall rings above and 762 cm (25 feet) of demister wire and 1524 cm (50 feet) of 1.59 cm (5/8") steel Pall rings below the catalyst bed. The catalyst was loaded into tubular 2.54 cm (one inch) wire casings positioned diagonally on demister wire and rolled into a bale of about 7.62 cm (3") diameter.

The hydrocarbon is fed to the column below the catalyst. To start up the overhead pressure is set to 928.13 KPa (120 psig) and the reboiler is charged with hydrocarbon fed at about 9.07 kg (20 pounds) per hour with the reboiler set at 10% which is maintained for 15 minutes at which time the feed rate is adjusted to maintain 50-75% bottoms level until the overhead temperature is within' 20 degrees of the bottoms temperature then increase the hydrocarbon feed rate to 45.4 kg/h (100 1b/hr). When the differential pressure reaches 6.89 KPa (1.0 psi) the hydrogen flow is initiated at 0.425 m³/h (15 scfh). Bottoms are heated to a uniform temperature of 71°C (160° F) then a mid relux flow is started. The overhead pressure is selected and the reaction distillation is carried out. The C₄ hydrocarbon feed conditions and results for each run are set out in TABLES I and II.

Runs carried out in the LPC mode removed all of the dienes whereas conventional distillation left a few parts per million under the same conditions. The internal reflux rate is reported as the ratio of the liquid immediately below the catalyst packing to the distillate (L/D). The data shows the LPC mode to give better diene removal.

**TABLE 1**

| EXAMPLE 1- RUN 1- Part 1 | | | | |
|---|---|---|---|---|
| HOURS ON LINE 41 | | | | |
| CONDITIONS | | | | |
| Hydrogen pp (psia) KPa | (2.3) 15.85 | | | |
| Feed Source | Refinery FCC | | | |
| Feed Rate (1b/hr) kg/h H₂ Rate (scfh) m³/h | (100) 45.4 (15) 0.425 | | | |
| Pressure, (psig) KPa | (120) 928.13 | | | |
| Distillate (1b/hr) kg/h | (93) 42.2 | | | |
| Internal Reflux Rate | 0.73 | | | |
| Mode | Conventional | | | |

| RESULTS | | | | |
|---|---|---|---|---|
| Analysis | | | | |
| | Feed wt % | Overhead wt % | Bottoms wt % | Vent wt % |
| Ethylene | 0.02 | 0.01 | 0.00 | 0.00 |
| Ethane | 0.20 | 0.01 | 0.00 | 0.69 |
| Propylene | 0.27 | 0.22 | 0.00 | 2.61 |
| Propane | 0.48 | 0.44 | 0.00 | 3.34 |
| Isobutane | 31.38 | 32.63 | 0.18 | 31.70 |
| Isobutene | 14.21 | 14.77 | 0.74 | 11.41 |
| Butene-1 | 12.82 | 4.59 | 1.07 | 3.25 |
| 1,3-Butadiene | 0.2788 | 0.0030 | 0.0199 | 0.0000 |
| N-Butane | 9.37 | 10.13 | 8.55 | 4.95 |
| Trans-butene-2 | 17.13 | 24.69 | 25.56 | 11.49 |
| 2,2-Dimethylpropane | 0.00 | 0.00 | 0.08 | |
| Methylcyclopropane | 0.02 | 0.02 | 0.02 | |
| Cis-butene-2 | 12.50 | 12.20 | 53.01 | 4.91 |
| C'₅'s | 1.26 | 0.27 | 10.58 | 0.06 |
| Heavies | 0.01 | 0.01 | 0.18 | 0.21 |
| Total | 100.00 | 100.0 | 100.0 | |
| (1bs/hr) kg/h | (100.0) 45.4 | (93.0) 42.2 | (5) 2.3 | (1.8) 0.8 |
| Temp. (°F) °C | | (155) 68 | (188) 87 | |

| COMPONENT SUMMARY | | | | |
|---|---|---|---|---|
| Component | Feed wt % | | Product wt % | |
| Dienes | 0.28 | | 30 ppm | |
| Isobutane | 31.4 | | 31.0 | |
| N-Butenes | 42.5 | | 43.0 | |
| N-Butane | 9.4 | | 10.0 | |
| % Butene-1 of total N-Butenes | 30.2 | | 10.2 | |

| EXAMPLE 1-RUN 1-part 2 | | | | |
|---|---|---|---|---|
| HOURS ON LINE 102 | | | | |
| CONDITIONS | | | | |
| Hydrogen pp (psia) KPa | (1.5) 10.34 | | | |
| Feed Source | Refinery FCC | | | |
| Feed Rate (1b/hr) kg/h | (100) 45.4 | | | |
| H₂ Rate (scfh) m³/h | (10) 0.283 | | | |
| Pressure, (psig) KPa | (120) 928.13 | | | |
| Distillate (1b/hr) kg/h | (93) 42.2 | | | |
| Internal Reflux Rate | 0.74 | | | |
| Mode | LPC | | | |

| RESULTS | | | | |
|---|---|---|---|---|
| Analysis | | | | |
| | Feed wt % | Overhead wt % | Bottoms wt % | Vent wt % |
| Ethylene | 0.02 | 0.01 | 0.00 | 0.00 |
| Ethane | 0.20 | 0.02 | 0.00 | 0.70 |
| Propylene | 0.27 | 0.25 | 0.00 | 2.13 |
| Propane | 0.48 | 0.47 | 0.00 | 2.52 |
| Isobutane | 31.38 | 32.97 | 0.21 | 23.22 |
| Isobutene | 14.21 | 14.82 | 0.81 | 8.18 |
| Butene-1 | 12.82 | 3.67 | 1.15 | 1.92 |
| 1,3-Butadiene | 0.2788 | 0.0000 | 0.0211 | 0.0000 |
| N-Butane | 9.37 | 9.93 | 8.84 | 3.43 |
| Trans-butene-2 | 17.13 | 25.37 | 25.71 | 8.29 |
| 2,2-Dimethylpropane | 0.00 | 0.00 | 0.08 | |
| Methylcyclopropane | 0.02 | 0.02 | 0.02 | |
| Cis-butene-2 | 12.50 | 12.20 | 52.25 | 3.46 |
| C'₅'s | 1.26 | 0.28 | 10.70 | 0.00 |
| Heavies | 0.01 | 0.00 | 0.20 | 0.00 |
| Total | 100.00 | 100.0 | 100.00 | |
| (1bs/hr) kg/h | (100.0) 45.4 | (93.1) 41.9 | (5) 2.3 | (2.0) 0.9 |
| Temp. (°F) °C | | (155) 68 | (188) 87 | |

| COMPONENT SUMMARY | | | | |
|---|---|---|---|---|
| Component | Feed wt % | | Product wt % | |
| Dienes | 0.28 | | 0 ppm | |
| Isobutane | 31.4 | | 31.2 | |
| N-Butenes | 42.5 | | 42.6 | |
| N-Butane | 9.4 | | 9.8 | |
| % Butene-1 of total N-Butenes | 30.2 | | 8.2 | |

| EXAMPLE 1-RUN 1-part 3 | | | | |
|---|---|---|---|---|
| HOURS ON LINE 161 | | | | |
| CONDITIONS | | | | |
| Hydrogen pp (psia) KPa | (0.8) 5.51 | | | |
| Feed Source | Refinery FCC | | | |
| Feed Rate (1b/hr) kg/h | (100) 45.4 | | | |
| H₂ Rate (scfh) m³/h | (5) 0.142 | | | |
| Pressure, (psig) KPa | (120) 928.13 | | | |
| Distillate (1b/hr) kg/h | (93) 42.2 | | | |
| Internal Reflux Rate | 0.74 | | | |
| Mode | Conventional | | | |

| RESULTS | | | | |
|---|---|---|---|---|
| Analysis | | | | |
| | Feed wt % | Overhead wt % | Bottoms wt % | Vent wt % |
| Ethylene | 0.02 | 0.01 | 0.00 | 0.00 |
| Ethane | 0.20 | 0.01 | 0.00 | 0.64 |
| Propylene | 0.27 | 0.25 | 0.00 | 1.96 |
| Propane | 0.48 | 0.46 | 0.00 | 2.24 |
| Isobutane | 31.38 | 32.84 | 0.47 | 20.08 |
| Isobutene | 14.21 | 14.87 | 0.87 | 7.12 |
| Butene-1 | 12.82 | 10.32 | 1.20 | 4.26 |
| 1,3-Butadiene | 0.2788 | 0.0262 | 0.0227 | 0.0000 |
| N-Butane | 9.37 | 9.52 | 8.71 | 2.87 |
| Trans-butene-2 | 17.13 | 19.35 | 25.66 | 5.71 |
| 2,2-Dimethylpropane | 0.00 | 0.00 | 0.08 | |
| Methylcyclopropane | 0.02 | 0.02 | 0.02 | |
| Cis-butene-2 | 12.50 | 12.08 | 52.60 | 2.96 |
| C₅'s | 1.26 | 0.24 | 10.23 | 0.00 |
| Heavies | 0.01 | 0.00 | 0.12 | 0.00 |
| Total | 100.00 | 100.0 | 100.00 | |
| (lbs/hr) kg/h | (100.0)45.4 | (92.7) 41.7 | (5) 2.3 | (2.3)1 |
| Temp. (°F) °C | | (156) 69 | (188) 87 | |

| COMPONENT SUMMARY | | | | |
|---|---|---|---|---|
| Component | Feed wt % | | Product wt % | |
| Dienes | 0.28 | | 262 ppm | |
| Isobutane | 31.4 | | 30.9 | |
| N-Butenes | 42.5 | | 43.0 | |
| N-Butane | 9.4 | | 9.3 | |
| % Butene-1 of total N-Butenes | 30.2 | | 22.6 | |

| EXAMPLE 1-RUN 1-part 4 | | | | |
|---|---|---|---|---|
| HOURS ON LINE 198 | | | | |
| CONDITIONS | | | | |
| Hydrogen pp (psia) KPa | (1.5) 10.34 | | | |
| Feed Source | Refinery FCC | | | |
| Feed Rate (1b/hr) kg/h | (100) 45.4 | | | |
| H₂ Rate (scfh) m³/h | (10) 0.283 | | | |
| Pressure, (psig) KPa | (120) 928.13 | | | |
| Distillate (1b/hr)kg/h | (93) 42.2 | | | |
| Internal Reflux Rate | 0.74 | | | |
| Mode | Conventional | | | |

| RESULTS | | | | |
|---|---|---|---|---|
| Analysis | | | | |
| | Feed wt % | Overhead wt % | Bottoms wt % | Vents wt % |
| Ethylene | 0.02 | 0.01 | 0.00 | 0.00 |
| Ethane | 0.20 | 0.01 | 0.02 | 0.64 |
| Propylene | 0.27 | 0.25 | 0.03 | 2.08 |
| Propane | 0.48 | 0.46 | 0.05 | 2.42 |
| Isobutane | 31.38 | 32.97 | 3.92 | 20.74 |
| Isobutene | 14.21 | 14.90 | 2.43 | 7.27 |
| Butene-1 | 12.82 | 7.29 | 2.61 | 3.26 |
| 1,3-Butadiene | 0.2788 | 0.0105 | 0.0509 | 0.0000 |
| N-Butane | 9.37 | 9.64 | 8.81 | 2.90 |
| Trans-butene-2 | 17.13 | 21.91 | 24.64 | 6.32 |
| 2,2-Dimethylpropane | 0.00 | 0.00 | 0.07 | |
| Methylcyclopropane | 0.02 | 0.02 | 0.02 | |
| Cis-butene-2 | 12.50 | 12.29 | 47.97 | 2.97 |
| C'₅'s | 1.26 | 0.23 | 9.22 | 0.00 |
| Heavies | 0.01 | 0.01 | 0.17 | 0.00 |
| Total | 100.00 | 100.0 | 100.00 | |
| (1bs/hr) kg/h | (100.0) 45.4 | (93.0) 41.9 | (5) 2.3 | (2.1) 0.9 |
| Temp. (°F) °C | | (155) 68 | (188) 87 | |

| COMPONENT SUMMARY | | | | |
|---|---|---|---|---|
| Component | Feed wt % | | Product wt % | |
| Dienes | 0.28 | | 105 ppm | |
| Isobutane | 31.4 | | 31.3 | |
| N-Butenes | 42.5 | | 42.6 | |
| N-Butane | 9.4 | | 9.5 | |
| % Butene-1 of total N-Butenes | 30.2 | | 16.4 | |

**TABLE II**

| EXAMPLE 1-RUN 2-part 1 | | | | |
|---|---|---|---|---|
| HOURS ON LINE 138 | | | | |
| CONDITIONS | | | | |
| Hydrogen pp (psia) KPa | (1.5) 10.34 | | | |
| Feed Source | Refinery FCC | | | |
| Feed Rate (1b/hr) kg/h | (56) 25.4 | | | |
| H₂ Rate (scfh) m³/h | (15) 0.425 | | | |
| Pressure, (psig) KPa | (125) 962.58 | | | |
| Distillate (1b/hr) kg/h | (37) 16.8 | | | |
| Internal Reflux Rate | 4.72 | | | |
| Mode | LPC | | | |

| RESULTS | | | | |
|---|---|---|---|---|
| Analysis | | | | |
| | Feed wt % | Overhead wt % | Bottoms wt % | Vent wt % |
| Ethylene | 0.03 | 0.03 | 0.00 | 0.00 |
| Ethane | 0.22 | 0.11 | 0.00 | 0.14 |
| Propylene | 0.24 | 0.31 | 0.00 | 0.68 |
| Propane | 0.53 | 0.68 | 0.00 | 1.18 |
| Isobutane | 28.17 | 39.45 | 0.01 | 33.16 |
| Isobutene | 15.05 | 21.03 | 0.0032 | 16.91 |
| Butene-1 | 13.91 | 7.81 | 0.01 | 5.99 |
| 1,3-Butadiene | 0.3441 | 0.0000 | 0.0000 | 0.0000 |
| N-Butane | 8.65 | 7.34 | 12.86 | 5.04 |
| Trans-butene-2 | 17.76 | 16.71 | 40.71 | 11.43 |
| 2,2-Dimethylpropane | 0.00 | 0.00 | 0.04 | |
| Methylcyclopropane | 0.02 | 0.01 | 0.03 | |
| Cis-butene-2 | 13.55 | 6.51 | 40.84 | 4.26 |
| C'₅'s | 1.53 | 0.02 | 5.47 | 0.00 |
| Heavies | 0.00 | 0.00 | 0.01 | 0.05 |
| Total | 100.00 | 100.0 | 100.00 | |
| (1bs/hr) kg/h | (56.0) 25.4 | (37.1) 16.7 | (15) 6.8 | (4.0) 1.8 |
| Temp. (°F) °C | | (164) 73 | (182)83 | |

| COMPONENT SUMMARY | | | | |
|---|---|---|---|---|
| Component | Feed wt % | | Product wt % | |
| Dienes | 0.3 | | 0 ppm | |
| Isobutane | 28.2 | | 28.5 | |
| N-Butenes | 45.2 | | 43.9 | |
| N-Butane | 8.7 | | 8.7 | |
| % Butene-1 of total N-Butenes | 30.8 | | 12.8 | |

| EXAMPLE 1-RUN 2-part 2 | | | | |
|---|---|---|---|---|
| HOURS ON LINE 173 | | | | |
| CONDITIONS | | | | |
| Hydrogen pp(psia) KPa | (1.5) 10.34 | | | |
| Feed Source | Refinery FCC | | | |
| Feed Rate (1b/hr) kg/h | (56) 25.4 | | | |
| H₂ Rate (scfh) m³/h | (15) 0.425 | | | |
| Pressure, (psig) KPa | (125) 962.58 | | | |
| Distillate (1b/hr) kg/h | (32) 14.5 | | | |
| Internal Reflux Rate | 5.46 | | | |
| Mode | LPC | | | |

| RESULTS | | | | |
|---|---|---|---|---|
| Analysis | | | | |
| | Feed wt % | Overhead wt % | Bottoms wt % | Vent wt % |
| Ethylene | 0.03 | 0.03 | 0.00 | 0.02 |
| Ethane | 0.22 | 0.09 | 0.00 | 0.29 |
| Propylene | 0.24 | 0.33 | 0.00 | 0.77 |
| Propane | 0.53 | 0.74 | 0.00 | 2.56 |
| Isobutane | 28.17 | 43.97 | 0.01 | 43.60 |
| Isobutene | 15.05 | 23.39 | 0.0541 | 5.45 |
| Butene-1 | 13.91 | 7.20 | 0.22 | 19.75 |
| 1,3-Butadiene | 0.3441 | 0.0000 | 0.0024 | 0.0000 |
| N-Butane | 8.65 | 5.24 | 15.64 | 3.88 |
| Trans-butene-2 | 17.76 | 13.92 | 42.32 | 8.80 |
| 2,2-Dimethylpropane | 0.00 | 0.00 | 0.03 | |
| Methylcyclopropane | 0.02 | 0.01 | 0.03 | |
| Cis-butene-2 | 13.55 | 5.07 | 37.33 | 3.22 |
| C'₅'s | 1.53 | 0.00 | 4.35 | 0.00 |
| Heavies | 0.00 | 0.00 | 0.01 | 0.00 |
| Total | 100.00 | 100.0 | 100.00 | |
| (1bs/hr) kg/h | (56.0) 25.4 | (32.2) 14.5 | (19) 8.6 | (4.9) 2.2 |
| Temp. (°F) °C | | (164) 73 | (182) 83 | |

| COMPONENT SUMMARY | | | | |
|---|---|---|---|---|
| Component | Feed wt % | | Product wt % | |
| Dienes | 0.3 | | 0 ppm | |
| Isobutane | 28.2 | | 29.1 | |
| N-Butenes | 45.2 | | 44.9 | |
| N-Butane | 8.7 | | 8.6 | |
| % Butene-1 of total N-Butenes | 30.8 | | 13.3 | |

### Example 2

### LIGHT FCC NAPHTHA STREAMS

The same procedures as set out in Example 1 were used, however the catalyst was G68C-1, a 0.4% palladium on 2.83mm-1.68mm Sieve opening (7-12 mesh) alumina product of United Catalyst, Inc. 914.4 cm (Thirty feet) of catalyst packing 0.0425 m³ (1.5 cubic foot) of catalytic material) prepared as a distillation structure as described in Example 1 was loaded in a 7.62 cm (three inch) column, with 152.4 cm (5 feet) of 1.59cm (5/8") steel pall rings and 304.8 cm (10 feet) of open space above and 91.44 cm (3 feet) of demister wire and 1524 cm (50 feet) of 1.59 cm (5/8") steel pall rings below. The distillation was conducted to take hydrotreated C₅'s overhead and the heavier components as bottoms. The feeds, conditions and results for each of three runs are set out in TABLES III - V.

The results of Run 3 at 74 hours on stream which was carried out at low hydrogen partial pressure and high 20 WHSV were below expectations. By increasing the hydrogen partial pressure to only 30.32 KPa (4.4 psia) at 272 hours on stream in a conventional distillation mode the diene removal was improved 10 fold.

**TABLE III**

| EXAMPLE 2 -RUN 1 | | | | |
|---|---|---|---|---|
| HOURS ON LINE 99 | | | | |
| CONDITIONS | | | | |
| Hydrogen pp, (psia) KPa | (4.5) 31 | | | |
| Feed Rate, (1b/hr) kg/h | (219) 99 | | | |
| H₂ Rate, (scfh) m³/h | (20) 0.566 | | | |
| Pressure, (psig) KPa | (125) 962.58 | | | |
| Distillate, (1b/hr) kg/h | (47) 21.3 | | | |
| Internal Reflux Rate | 2.11 | | | |
| Mode | LPC | | | |

| RESULTS | | | | |
|---|---|---|---|---|
| Analysis | | | | |
| | Feed wt % | Overhead wt % | Bottoms wt % | Vent wt % |
| C₄'s | 0.00 | 0.00 | 0.00 | |
| Isobutane | 0.00 | 0.02 | 0.00 | 0.02 |
| Other C₄'s | 0.33 | 1.52 | 0.04 | |
| 3-Methyl Butene-1 | 0.28 | 0.27 | 0.06 | |
| Isopentane | 7.40 | 32.40 | 2.32 | 33.23 |
| Pentene-1 | 1.07 | 1.47 | 0.34 | |
| 2-Methyl Butene-1 | 1.81 | 4.09 | 0.60 | |
| N-Pentane | 3.82 | 17.65 | 1.62 | |
| 2-Methyl Butadiene-1,3 | 0.1761 | 0.0000 | 0.0595 | |
| Trans-Pentene-2 | 2.45 | 14.99 | 1.02 | |
| Unknown 1 | 0.02 | 0.04 | 0.02 | |
| Cis-Pentene-2 | 1.36 | 4.86 | 0.59 | 21.62 |
| 2-Methyl Butene-2 | 3.28 | 17.93 | 1.69 | |
| Trans-Piperylene | 0.18 | 0.00 | 0.10 | |
| Cis-Piperylene | 0.06 | 0.00 | 0.04 | |
| Cyclopentene | 0.29 | 0.26 | 0.26 | |
| Unknowns 2 | 5.12 | 4.12 | 11.26 | |
| Heavies | 72.36 | 0.38 | 79.97 | |
| Total | 100.00 | 100.0 | 100.00 | |
| (1bs/hr) kg/h | (218.8) 98.5 | (47.0) 21.3 | (171.9)77.4 | 0.00 |
| Temp. (°F) °C | | (256) 124 | (417) 214 | |

| COMPONENT SUMMARY | | | | |
|---|---|---|---|---|
| Component | Feed wt % | | Product wt % | |
| Dienes | 1.91 | | 17 ppm | |
| 2MB1 + 2MB2 | 22.9 | | 24.2 | |
| Isopentane | 33.4 | | 32.5 | |
| Pentenes | 22.0 | | 22.6 | |
| N-Pentane | 17.2 | | 18.8 | |
| % 3-MB-1 of total Isoamylenes | 5.23 | | 1.63 | |
| % Pentene-1 of total N-Pentenes | 22.0 | | 9.5 | |

**TABLE IV**

| EXAMPLE 2-RUN 2-page 1 | | | | |
|---|---|---|---|---|
| HOURS ON LINE 45 | | | | |
| CONDITIONS | | | | |
| Hydrogen pp, (psia) KPa | (4.8) 33.1 | | | |
| Feed Rate, (1b/hr) kg/h | (217) 98 | | | |
| H₂ Rate, (scfh) m³/h | (20) 0.566 | | | |
| Pressure, (psig) KFa | (125) 962.58 | | | |
| Distillate, (1b/hr) kg/h | (42) 19.1 | | | |
| Internal Reflux Rate | 2.22 | | | |
| Mode | LPC | | | |

| RESULTS | | | | |
|---|---|---|---|---|
| Analysis | | | | |
| | Feed wt % | Overhead wt % | Bottoms wt % | Vent wt % |
| C₄'s | 0.00 | 0.00 | 0.00 | |
| Isobutane | 0.03 | 0.16 | 0.00 | 0.32 |
| Other C₄'s | 1.46 | 7.33 | 0.00 | |
| 3-Methyl Butene-1 | 0.21 | 0.20 | 0.00 | |
| Isopentane | 7.85 | 38.92 | 0.08 | 34.49 |
| Pentene-1 | 0.76 | 1.04 | 0.02 | |
| 2-Methyl Butene-1 | 1.37 | 3.08 | 0.06 | |
| N-Pentane | 3.56 | 13.79 | 0.73 | |
| 2-Methyl Butadiene-1,3 | 0.101 | 0.0000 | 0.0110 | |
| Trans-Pentene-2 | 2.08 | 10.77 | 0.42 | |
| Unknown 1 | 0.02 | 0.04 | 0.01 | |
| Cis-Pentene-2 | 1.15 | 3.50 | 0.29 | 16.97 |
| 2-Methyl Butene-2 | 2.87 | 13.90 | 0.91 | |
| Trans-Piperylene | 0.11 | 0.00 | 0.04 | |
| Cis-Piperylene | 0.04 | 0.00 | 0.01 | |
| Cyclo-C₅ | 0.31 | 0.43 | 0.23 | |
| Unknowns 2 | 6.49 | 6.01 | 6.46 | |
| Heavies | 71.58 | 0.83 | 90.71 | |
| Total | 100.00 | 100.00 | 100.00 | |
| (1bs/hr ) kg/h | (217.3) 97.8 | (41.9) 18.9 | (175.4)78.9 | 0.00 |
| Temp. (°F) °C | | (260) 127 | (406) 208 | |

| COMPONENT SUMMARY | | | | |
|---|---|---|---|---|
| Component | Feed wt % | | Product wt % | |
| Dienes | 1.21 | | 0 ppm | |
| 2MB1 + 2MB2 | 20.8 | | 21.6 | |
| Isopentane | 38.4 | | 40.3 | |
| Pentenes | 19.6 | | 18.9 | |
| N-Pentane | 17.4 | | 17.3 | |
| % 3-MB-1 of total Isoamylenes | 4.80 | | 0.94 | |
| % Pentene-1 of total N-Pentenes | 19.0 | | 6.1 | |

| EXAMPLE 2-RUN 2-page 2 | | | | |
|---|---|---|---|---|
| HOURS ON LINE 201 | | | | |
| CONDITIONS | | | | |
| Hydrogen pp (psia) KPa | (2.9) 19.98 | | | |
| Feed Rate, (1b/hr) kg/h | (219) 99 | | | |
| H₂ Rate, (scfh) m³/h | (20) 0.566 | | | |
| Pressure, (psig) KPa | (75) 618.08 | | | |
| Distillate, (1b/hr) kg/h | (41) 18.6 | | | |
| Internal Reflux Rate | 2.50 | | | |
| Mode | LPC | | | |

| RESULTS | | | | |
|---|---|---|---|---|
| Analysis | | | | |
| | Feed wt % | Overhead wt % | Bottoms wt % | Vents wt % |
| C₄'s | 0.00 | 0.02 | 0.00 | |
| Isobutane | 0.03 | 0.15 | 0.00 | 0.30 |
| Other C₄'s | 1.48 | 6.85 | 0.00 | |
| 3-Methyl Butene-1 | 0.21 | 0.32 | 0.00 | |
| Isopentane | 7.85 | 36.28 | 0.03 | 42.53 |
| Pentene-1 | 0.76 | 1.18 | 0.01 | |
| 2-Methyl Butene-1 | 1.37 | 3.94 | 0.04 | |
| N-Pentane | 3.56 | 15.98 | 0.58 | |
| 2-Methyl Butadiene-1,3 | 0.1013 | 0.0000 | 0.0085 | |
| Trans-Pentene-2 | 2.08 | 12.08 | 0.33 | |
| Unknown 1 | 0.02 | 0.05 | 0.00 | |
| Cis-Pentene-2 | 1.15 | 3.88 | 0.23 | 18.14 |
| 2-Methyl Butene-2 | 2.87 | 13.81 | 0.76 | |
| Trans-Piperylene | 0.11 | 0.00 | 0.04 | |
| Cis-Piperylene | 0.04 | 0.00 | 0.01 | |
| Cyclo-C₅ | 0.31 | 0.42 | 0.20 | |
| Unknowns 2 | 6.49 | 4.49 | 6.35 | |
| Heavies | 71.58 | 0.54 | 91.42 | |
| Total | 100.00 | 100.00 | 100.00 | |
| (1bs/hr) kg/h | (219.0) 99 | (41.0) 18.6 | (175.7) 79.1 | (2.6) 1.2- |
| Temp. (°F) °C | | (212) 100 | (359) 182 | |

| COMPONENT SUMMARY | | | | |
|---|---|---|---|---|
| Component | Feed wt % | | Product wt % | |
| Dienes | 1.21 | | 0 ppm | |
| 2MB1 + 2MB2 | 20.8 | | 20.9 | |
| Isopentane | 38.4 | | 38.6 | |
| Pentenes | 19.6 | | 20.5 | |
| N-Pentane | 17.4 | | 18.2 | |
| % 3-MB-1 of total Isoamylenes | 4.80 | | 1.50 | |
| % Pentene-1 of total N-Pentenes | 19.0 | | 6.0 | |

**TABLE V**

| EXAMPLE 2-RUN 3-page 1 | | | | |
|---|---|---|---|---|
| HOURS ON LINE 44 | | | | |
| CONDITIONS | | | | |
| Hydrogen pp, (psia) KPa | (2.7) 18.6 | | | |
| Feed Rate, (1b/hr) kg/h | (295) 134 | | | |
| H₂ Rate, (scfh) m³/h | (20) 0.566 | | | |
| Pressure, (psig) KPa | (100) 790.33 | | | |
| Distillate, (1b/hr) kg/h | (55) 24.9 | | | |
| Internal Reflux Rate | 2.65 | | | |
| Mode | LPC | | | |

| RESULTS | | | | |
|---|---|---|---|---|
| Analysis | | | | |
| | Feed wt % | Overhead wt % | Bottoms wt % | Vent wt % |
| C₄'s | 0.02 | 0.07 | 0.00 | |
| Isobutane | 0.07 | 0.27 | 0.00 | 0.49 |
| Other C₄'s. | 1.41 | 0.00 | 0.00 | |
| 3-Methyl Butene-1 | 0.25 | 0.73 | 0.00 | |
| Isopentane | 8.66 | 36.35 | 0.20 | 26.57 |
| Pentene-1 | 0.87 | 2.09 | 0.02 | |
| 2-Methyl Butene-1 | 1.83 | 6.60 | 0.05 | |
| N-Pentane | 1.51 | 6.42 | 0.12 | |
| 2-Methyl Butadiene-1,3 | 0.0537 | 0.0024 | 0.0000 | |
| Trans-Pentene-2 | 2.58 | 13.00 | 0.21 | |
| Unknown 1 | 0.03 | 0.07 | 0.02 | |
| Cis-Pentene-2 | 1.42 | 5.13 | 0.22 | 28.45 |
| 2-Methyl Butene-2 | 3.93 | 15.45 | 1.12 | |
| Trans-Piperylene | 0.06 | 0.00 | 0.02 | |
| Cis-Piperylene | 0.03 | 0.00 | 0.01 | |
| Cyclo-C₅ | 0.06 | 0.05 | 0.04 | |
| Unknowns 2 | 7.50 | 6.65 | 7.69 | |
| Heavies | 69.71 | 7.12 | 90.28 | |
| Total | 100.00 | 100.00 | 100.00 (236.9) | |
| (1bs/hr) kg/h | (294.9) 132.7 | (55.0) 24.9 | 106.6 (3-1) | 1.4 |
| Temp. (°F) °C | | (240) 116 | (388) 198 | |

| COMPONENT SUMMARY | | | | |
|---|---|---|---|---|
| Component | Feed wt % | | Product wt % | |
| Dienes | 0.68 | | 53 ppm | |
| 2MB1 + 2MB2 | 27.1 | | 27.7 | |
| Isopentane | 40.7 | | 39.7 | |
| Pentenes | 22.9 | | 24.4 | |
| N-Pentane | 7.1 | | 7.1 | |
| % 3-MB-1 of total Isoamylenes | 4.23 | | 2.61 | |
| % Pentene-1 of total N-Pentenes | 17.8 | | 9.2 | |

| EXAMPLE 2-RUN 3-page 2 | | | | |
|---|---|---|---|---|
| HOURS ON LINE 74 | | | | |
| CONDITIONS | | | | |
| Hydrogen pp, (psia) KPa | (1.5) 10.34 | | | |
| Feed Rate, (1b/hr) kg/h | (295) 134 | | | |
| H₂ Rate, (scfh) m³/h | (10) 0.283 | | | |
| Pressure, (psig) KPa | (100) 790.33 | | | |
| Distillate, (1b/hr)kg/h | (52) 23.6 | | | |
| Internal Reflux Rate | 2.56 | | | |
| Mode | LPC | | | |

| RESULTS | | | | |
|---|---|---|---|---|
| Analysis | | | | |
| | Feed wt % | Overhead wt % | Bottoms wt % | Vent wt % |
| C₄'s | 0.02 | 0.08 | 0.00 | |
| Isobutane | 0.07 | 0.29 | 0.00 | 0.68 |
| Other C₄'s | 1.41 | 6.44 | 0.00 | |
| 3-Methyl Butene-1 | 0.25 | 1.13 | 0.00 | |
| Isopentane | 8.66 | 39.14 | 0.20 | 32.86 |
| Pentene-1 | 0.87 | 3.65 | 0.02 | |
| 2-Methyl Butene-1 | 1.83 | 8.35 | 0.05 | |
| N-Pentane | 1.51 | 5.84 | 0.12 | |
| 2-Methyl Butadiene-1,3 | 0.0537 | 0.0417 | 0.0000 | |
| Trans-Pentene-2 | 2.58 | 10.45 | 0.21 | |
| Unknown 1 | 0.03 | 0.07 | 0.02 | |
| Cis-Pentene-2 | 1.42 | 5.11 | 0.22 | 0.00 |
| 2-Methyl Butene-2 | 3.93 | 13.09 | 1.12 | |
| Trans-Piperylene | 0.06 | 0.03 | 0.02 | |
| Cis-Piperylene | 0.03 | 0.05 | 0.01 | |
| Cyclo-C₅ | 0.06 | 0.06 | 0.04 | |
| Unknowns 2 | 7.50 | 5.29 | 7.69 | |
| Heavies | 69.71 | 0.90 | 90.28% | |
| Total | 100.00 | 100.00 | 100.00 | |
| (1bs/hr) kg/h | (295.1) 134 | (52.0) 23.6 | (241.2) 108.5 (2.0) | 0.9 |
| Temp. (°F) °C | | (236) 113 | (383) 195 | |

| COMPONENT SUMMARY | | | | |
|---|---|---|---|---|
| Component | Feed wt % | | Product wt % | |
| Dienes | 0.68 | | 1263 ppm | |
| 2MB1 + 2MB2 | 27.1 | | 27.5 | |
| Isopentane | 40.7 | | 42.4 | |
| Pentenes | 22.9 | | 21.9 | |
| N-Pentane | 7.1 | | 6.5 | |
| % 3-MB-1 of total Isoamylenes | 4.23 | | 4.02 | |
| % Pentene-1 of total N-Pentenes | 17.8 | | 17.6 | |

| EXAMPLE 2-RUN 3-page 3 | | | | |
|---|---|---|---|---|
| HOURS ON LINE 272 | | | | |
| CONDITIONS | | | | |
| Hydrogen pp, (psia) KPa | (4.4) 30.32 | | | |
| Feed Rate, (1b/hr) kg/h | (295) 134 | | | |
| H₂ Rate, (scfh) m³/h | (30) 0.850 | | | |
| Pressure, (psig) KPa | (100) 790.33 | | | |
| Distillate, (1b/hr) kg/h | (53) 24 | | | |
| Internal Reflux Rate | 2.43 | | | |
| Mode | Conventional | | | |

| RESULTS | | | | |
|---|---|---|---|---|
| Analysis | | | | |
| | Feed wt % | Overhead wt % | Bottoms wt % | Vents wt % |
| C₄'s | 0.02 | 0.08 | 0.00 | |
| Isobutane | 0.07 | 1.03 | 0.00 | 1.58 |
| Other C₄'s | 1.41 | 11.21 | 0.00 | |
| 3-Methyl Butene-1 | 0.25 | 0.91 | 0.00 | |
| Isopentane | 8.66 | 42.05 | 0.60 | 26.14 |
| Pentene-1 | 0.87 | 2.11 | 0.14 | |
| 2-Methyl Butene-1 | 1.83 | 6.24 | 0.36 | |
| N-Pentane | 1.51 | 5.06 | 0.63 | |
| 2-Methyl Butadiene-1,3 | 0.0537 | 0.0057 | 0.0000 | |
| Trans-Pentene-2 | 2.58 | 9.32 | 0.98 | |
| Unknown 1 | 0.03 | 0.06 | 0.02 | |
| Cis-Pentene-2 | 1.42 | 3.98 | 0.60 | 18.29 |
| 2-Methyl Butene-2 | 3.93 | 11.56 | 1.87 | |
| Trans-Piperylene | 0.06 | 0.00 | 0.03 | |
| Cis-Piperylene | 0.03 | 0.01 | 0.01 | |
| Cyclo-C₅ | 0.06 | 0.04 | 0.04 | |
| Unknowns 2 | 7.50 | 5.29 | 13.06 | |
| Heavies | 69.71 | 1.11 | 81.65 | |
| Total | 100.00 | 100.00 | 100.00 | |
| (1bs/hr) kg/h Temp. (°F) °C | (295.0) 134 | (53.0) 24 (233) 112 | (238.1) 107.2 (4.00) (369) 187 | 1.8 |

| COMPONENT SUMMARY | | | | |
|---|---|---|---|---|
| Component | Feed wt % | | Product wt % | |
| Dienes | 0.68 | | 126 ppm | |
| 2MB1 + 2MB2 | 27.1 | | 25.7 | |
| Isopentane | 40.7 | | 43.1 | |
| Pentenes | 22.9 | | 22.6 | |
| N-Pentane | 7.1 | | 7.3 | |
| % 3-MB-1 of total Isoamylenes | 4.23 | | 3.17 | |
| % Pentene-1 of total N-Pentenes | 17.8 | | 11.2 | |

### EXAMPLE 3

### C₃ STREAMS

The same procedures as set out in Example 1 were used, however the catalyst in Run 1 was G68C, a 0.3% palladium on 6.73mm-3.36mm Sieve opening (3-6 mesh) alumina product of United Catalyst, Inc. Twenty feet of catalyst packing (0.0283 m³ 1.0 cubic foot) of catalytic material) prepared as distillation structures as described in U.S Pat No. 5,266,546, which are tubular wire mesh about 5.08 cm (2") long with 5.08 cm (2") diameter having the ends sealed at 90° to each other. The catalyst packing was loaded in a 7.62 cm (three inch) column, with 152.4 cm (5 feet) of 7.59 cm (5/8") steel pall rings and 304.8 cm (10 feet) of open space above and 91.44 cm (3 feet) of demister wire and 1524 cm (50 feet) of 1.59 cm (5/8") steel pall rings below. The same structures and column was used in Run 2, but the catalyst was United Catalyst G68H (0.3% Pd and 0.3% Ag on alumina)

The distillation was conducted to take hydrotreated C₅'s overhead and the heavier components as bottoms. The feeds, conditions and results for each of three runs are set out in TABLES VI and VII.

**TABLE VI**

| EXAMPLE 3-RUN 1 | | | | |
|---|---|---|---|---|
| HOURS ON LINE 213 | | | | |
| CONDITIONS | | | | |
| Hydrogen pp, (psia) KPa | (6.4) 44.1 | | | |
| Feed Rate, (1b/hr) kg/h | (90) 40.8 | | | |
| H₂ Rate, (scfh) m³/h | (34) 0.963 | | | |
| Pressure, (psig) KPa | (250) 1823.83 | | | |
| Distillate, (1b/hr) kg/h | (82) 37.2 | | | |
| Internal Reflux Ratio | 0.88 | | | |
| Mode | LPC | | | |

| RESULTS | | | | |
|---|---|---|---|---|
| Analysis | | | | |
| | Feed wt % | Overhead wt % | Bottoms wt % | Vent wt % |
| Methane | 0.00 | 0.00 | 0.00 | 0.06 |
| Ethylene | 0.00 | 0.00 | 0.00 | 0.45 |
| Ethane | 0.05 | 0.03 | 0.00 | |
| Propene | 85.43 | 88.88 | 22.85 | 78.94 |
| Propane | 9.93 | 11.02 | 8.81 | 7.20 |
| Methylacetylene | 1.99 | 0.0000 | 7.66 | |
| Propadiene | 0.81 | 0.0000 | 5.45 | |
| Cyclopropane | 0.05 | 0.04 | 0.34 | |
| Isobutane | 0.00 | 0.00 | 0.10 | |
| Isobutene | 0.01 | 0.00 | 0.27 | |
| Butene-1 | 0.21 | 0.00 | 6.30 | 0.13 |
| Butadiene | 1.37 | 0.00 | 25.59 | |
| N-butane | 0.00 | 0.00 | 2.56 | |
| Vinylacetylene | 0.00 | 0.00 | 0.22 | |
| Trans-butene-2 | 0.01 | 0.00 | 2.34 | |
| Cis-butene-2 | 0.01 | 0.00 | 1.12 | |
| C₅'s | 0.04 | 0.00 | 0.75 | 0.04 |
| C₆'s | 0.00 | 0.00 | 13.51 | |
| Heavies | 0.00 | 0.00 | 0.01 | 0.05 |
| Total | 100.00 | 100.0 | 100.00 | |
| (1bs/hr) kg/h | (56.0) 25.4 | (37.1) 16.7 | (15) 6.8 (4.1) | 1.9 |
| Temp. (°F) °C | | (113) 45 | (216) 102 | |

| COMPONENT SUMMARY | | | | |
|---|---|---|---|---|
| Component | Feed wt % | | Product wt % | |
| Dienes | 4.2 | | 0 ppm | |
| Propylene | 85.4 | | 85.8 | |
| Propane | 9.9 | | 10.8 | |
| C₆ | 0.06 | | 0.65 | |

**TABLE VII**

| EXAMPLE 3-RUN 2 | | | | |
|---|---|---|---|---|
| HOURS ON LINE 152 | | | | |
| CONDITIONS | | | | |
| Hydrogen pp, (psia) KPa | (5.2) 35.83 | | | |
| Feed Rate, (lb/hr) kg/h | (90) 40.8 | | | |
| H₂ Rate, (scfh) m³/h | (34) 0.963 | | | |
| Pressure, (psig) KPa | (250) 1823.83 | | | |
| Distillate, (1b/hr) kg/h | (84) 38.1 | | | |
| Internal Reflux Ratio | 1.27 | | | |
| Mode | LPC | | | |

| RESULTS | | | | |
|---|---|---|---|---|
| Analysis | | | | |
| | Feed wt % | Overhead wt % | Bottoms wt % | Vent wt % |
| Methane | 0.00 | 0.00 | 0.00 | 0.01 |
| Ethylene | 0.00 | 0.00 | 0.00 | 0.54 |
| Ethane | 0.05 | 0.03 | 0.00 | |
| Propene | 85.43 | 88.94 | 22.17 | 74.14 |
| Propane | 9.93 | 10.12 | 10.13 | 6.47 |
| Methylacetylene | 1.99 | 0.0000 | 8.70 | |
| Propadiene | 0.81 | 0.0000 | 5.90 | |
| Cyclopropane | 0.05 | 0.03 | 0.43 | |
| Isobutane | 0.00 | 0.00 | 0.39 | |
| Isobutene | 0.01 | 0.00 | 0.34 | |
| Butene-1 | 0.21 | 0.00 | 6.08 | 0.13 |
| Butadiene | 1.37 | 0.00 | 30.57 | |
| N-butane | 0.00 | 0.00 | 3.02 | |
| Vinylacetylene | 0.00 | 0.00 | 0.25 | |
| Trans-butene-2 | 0.01 | 0.00 | 0.70 | |
| Cis-butene-2 | 0.01 | 0.00 | 0.22 | |
| C₅'s | 0.04 | 0.00 | 0.83 | 0.04 |
| C₆'s | 0.00 | 0.00 | 8.76 | |
| Heavies | 0.05 | 0.02 | 1.52 | |
| Total | 100.00 | 100.0 | 100.00 | |
| (1bs/hr) kg/h | (90.0)40.8 | (83.9)37.8 | (3.5)1.8 | (2.8)1.3 |
| Temp. (°F) °C | | (113) 45 | (171) 77 | |

| COMPONENT SUMMARY | | | | |
|---|---|---|---|---|
| Component | Feed wt % | | Product wt % | |
| Dienes | 4.2 | | 0 ppm | |
| Propylene | 85.4 | | 86.1 | |
| Propane | 9.9 | | 10.0 | |
| C₆ | 0.06 | | 0.42 | |

## Claims

1. A process for the selective hydrogenation of highly unsaturated compounds comprising feeding a hydrocarbon stream containing monoolefins together with highly unsaturated compounds which comprise diolefins and acetylenes along with a hydrogen stream at an effectuating hydrogen partial pressure in the'range of least (0.1 psia) 0.69 KPa to less than (10 psia) 68.9 KPa to a distillation column reactor containing a hydrogenation catalyst which is a component of a distillation structure, selectively hydrogenating a portion of the highly unsaturated compounds and operating at an internal reflux in the range of 0.2 to 20 L/D.

2. The process according to claim 1 wherein said hydrocarbon stream comprises aliphatic compounds having three to nine carbon atoms.

3. The process according to claim 1 wherein said hydrocarbon stream comprises a major amount of normal olefins which are recovered as products from said hydrogenation.

4. The process according to claim 1 wherein said hydrocarbon has a WHSV in the range of 0.1 to 35.

5. The process according to claim 2 wherein the overhead pressure is in the range of (0 to 350 psig) 0 to 2512.83 KPa.

6. The process according to claim 1 wherein the hydrogen partial pressure is less than (7 psia) 48.23 KPa.

7. The process according to claim 5 wherein the hydrogen partial pressure is less than (7 psia) 48.23 KPa.

8. The process according to claim 1 wherein hydrogenation catalyst comprises a Group VIII metal or metal compound as the principal catalytic component.

9. The process according to claim 8 wherein said catalyst comprises Pd.

10. The process according to claim 5 wherein said overhead pressure is in the range of (50 to 150 psia) 344.5 to 1033.5 KPa.

11. A process for the selective hydrogenation of highly unsaturated compounds comprising monoolefins, di-olefins and acetylenic compounds contained within a olefin rich stream, comprising the steps of:
(a) feeding (1) a first stream comprising olefins, diolefins and acetylenic hydrocarbon compounds and (2) a second stream containing hydrogen to a distillation column reactor into a feed zone;
(b) concurrently in said distillation column reactor under a hydrogen partial pressure of (0.1 psi) 0.69 KPa to less than (10 psia) 68.9 KPa and at an internal reflux in the range of 0.2 to 20 L/D:
(i) contacting said streams in a distillation reaction zone with a hydrogenation catalyst prepared in a form to act as a distillation structure thereby reacting essentially all of said di-olefins and acetylenic compounds with said hydrogen to form less unsaturated hydrocarbons in a reaction mixture, and
(ii) separating the olefins contained in said first stream and any olefins produced by said hydrogenation from said reaction mixture by fractional distillation.

12. The process according to claim 11 wherein said first steam is fed at or below the lower end of said distillation reaction zone.

13. The process according to claim 11 wherein said streams are fed separately to said distillation column reactor.

14. The process according to claim ,11 wherein said first and second streams are mixed prior to entry into said distillation column reactor

15. The process according to claim 11 wherein said hydrocarbon stream comprises aliphatic compounds having three to nine carbon atoms.

16. The process according to claim 15 wherein said hydrocarbon stream comprises a C₃ fraction.

17. The process according to claim 15 wherein said hydrocarbon stream comprises a C₄ cut.

18. The process according to claim 15 wherein said hydrocarbon stream comprises a C₅ cut.

19. The process according to claim 15 wherein said hydrocarbon stream comprises a C₆ cut.

20. The process according to claim 15 wherein said first and second streams are combined before feeding to said distillation column reactor.

21. The process according to claim 11 wherein hydrogenation catalyst comprises a Group VIII metal or metal compound as the principal catalytic component.

22. The process according to claim 21 wherein said hydrogenation catalyst comprises 0.1 to 5.0 wt% palladium oxide on alumina extrudates.

23. The process according to claim 11 wherein the overhead pressure of said distillation column reactor is between (240 and 315 psig) 1754.93 and 2271.68 KPa.

24. The process according to claim 23 wherein said first stream comprises propylene.

25. The process according to claim 11 wherein said distillation structure comprises flexible, semi-rigid open mesh tubular material wire mesh, filed with a particulate hydrogenation catalytic material.

26. The process according to claim 11 further comprising step (c) withdrawing the separated olefins from step (b) (ii) along with any alkanes and lighter compounds, including any unreacted hydrogen, from said distillation column reactor as overheads.

27. The process according to claim 11 wherein said distillation column reactor operates at overhead pressure of in the range between (0 and 250 psig) 0 and 1823.83 KPa.

28. The process according to claim 27 wherein said pressure is in the range of(50 to 150 psig) 445.83 to 1134.83 KPa.

29. The process according to claim 11 wherein temperatures within said distillation reaction zone are in the range of (40 to 300°F) 4.44 to 149 °C.

30. The process according to claim 29 wherein temperatures within said distillation reaction zone are in the range of (110 to 270°F) 43 to 132 °C.

31. The process according to claim 11 wherein substantially all of said di-olefins and acetylenic compounds are eliminated from said hydrocarbon stream.

32. The process according to claim 11 wherein the first stream has a WHSV in the range of 0.1 to 35.

33. The process according to claim 12 wherein hydrogenation catalyst comprises a Group VIII metal or metal compound as the principal catalytic component.

34. The process according to claim 33 wherein said hydrogenation catalyst comprises 0.1 to 5.0 wt% palladium oxide on alumina extrudates.

35. The process according to claim 33 wherein said distillation column reactor operates at overhead pressure of in the range between (0 and 250 psig) 0 and 1823.83 KPa.

36. The process according to claim 35 wherein temperatures within said distillation reaction zone are in the range of(40 to 300°F) 4.44 to 149°C.

37. The process according to claim 36 wherein said pressure is in the range of (50 to 150 psig) 445.83 to 1134.83 KPa.

38. The process according to claim 37 wherein temperatures within said distillation reaction zone are in the range of (110 to 270°F) 43 to 132°C.

39. The process according to claim 38 wherein the hydrogen partial pressure os in the range of (0.1 to 7 psia) 0.69 to 48.23 KPa.

40. The process according to claim 39 wherein there is internal reflux in the range of 0.5 to 5 L/D.

41. The process according to claim 11 wherein substantially all of the highly unsaturated compounds are hydrogenated.

42. A process for the isomerization of olefins comprising feeding a hydrocarbon stream containing olefins along with a hydrogen stream at an effectuating hydrogen partial pressure in the range of least (0.1 psia) 0.69 KPa to less than (10 psia) 68.9 KPa to a distillation column reactor containing a hydrogenation catalyst which is a component of a distillation structure and isomerizing a portion of the isomerizable olefins and operating at an internal reflux in the range of 0.2 to 20 L/D.

43. The process according to claim 42 wherein said hydrocarbon stream comprises aliphatic compounds having four to nine carbon atoms.

44. The process according to claim 42 wherein said hydrocarbon has a WHSV in the range of 0.1 to 35.

45. The process according to claim 43 wherein the overhead pressure is in the range of (0 to 350 psig) 0 to 2512.83 KPa.

46. The process according to claim 42 wherein the hydrogen partial pressure is less than ( 7 psia) 48.23 KPa.

47. The process according to claim 42 wherein the hydrogenation catalyst comprises a Group VIII metal or metal compound as the principal catalytic component.

48. The process according to claim 47 wherein said catalyst comprises Pd.

49. The process according to claim 45 wherein said overhead pressure is in the range of (50 to 150 psia) 344.5 to 1033.5 KPa.

50. The process according to claim 42 wherein said hydrocarbon stream comprises a light naphtha.

51. The process according to claim 50 wherein said light naphtha comprises 3-methyl butene-1.

52. The process according to claim 51 wherein a portion of 3-methyl butene-1 is isomerized to 2-methyl butene-2 and 2-methyl butene-1.

## Patentansprüche

1. Verfahren zur selektiven Hydrierung von hoch ungesättigten Verbindungen, umfassend die Zufuhr eines Kohlenwasserstoffstroms, enthaltend Monoolefine zusammen mit hoch ungesättigten Verbindungen, die Diolefine und Acetylene umfassen, zusammen mit einem Wasserstoffstrom bei einem Betriebswasserstoffpartialdruck im Bereich von zumindest (0,1 psia)0,69 KPa bis zu weniger als (10 psia) 68,9 KPa zu einem Destillationskolonnenreaktor, der einen Hydrierungskatalysator enthält, welcher eine Komponente einer Destillationsstruktur ist, die selektive Hydrierung eines Teils der hoch ungesättigten Verbindungen und die Durchführung bei einem inneren Rückfluß im Bereich von 0,2 bis 20 L/D.

2. Verfahren gemäß Anspruch 1, worin der Kohlenwasserstoffstrom aliphatische Verbindungen mit drei bis neun Kohlenstoffatomen umfaßt.

3. Verfahren gemäß Anspruch 1, worin der Kohlenwasserstoffstrom einen überwiegenden Anteil an normalen Olefinen umfaßt, die als Produkte aus der Hydrierung gewonnen werden.

4. Verfahren gemäß Anspruch 1, worin der Kohlenwasserstoff eine WHSV im Bereich von 0,1 bis 35 besitzt.

5. Verfahren gemäß Anspruch 2, worin der Überkopfdruck im Bereich von (0 bis 350 psig) 0 bis 2512,83 KPa liegt.

6. Verfahren gemäß Anspruch 1, worin der Wasserstoffpartialdruck geringer ist als (7 psia) 48,23 KPa.

7. Verfahren gemäß Anspruch 5, worin der Wasserstoffpartialdruck geringer ist als (7 psia) 48,23 KPa.

8. Verfahren gemäß Anspruch 1, worin der Hydrierungskatalysator eine Verbindung eines Metalls der Gruppe VIII oder eine Gruppe VIII-Metallverbindung als hauptsächliche katalytische Komponente umfaßt.

9. Verfahren gemäß Anspruch 8, worin der Katalysator Pd umfaßt.

10. Verfahren gemäß Anspruch 5, worin der Überkopfdruck im Bereich von (50 bis 150 psia) 344,5 bis 1033,5 KPa liegt.

11. Verfahren zur selektiven Hydrierung von hoch ungesättigten Verbindungen, umfassend Monoolefine, Diolefine und acetylenische Verbindungen, die enthalten sind in einem olefinreichen Strom, umfassend die Stufen:
(a) Zufuhr (1) eines ersten Stroms, umfassend Olefine, Diolefine und acetylenische Kohlenwasserstoffverbindungen, und (2) eines zweiten Stroms, enthaltend Wasserstoff, zu einem Destillationskolonnenreaktor in eine Beschickungszone;
(b) gleichzeitig in diesem Destillationskolonnenreaktor unter einem Wasserstoffpartialdruck von (0,1 psi) 0,69 KPa bis weniger als (10 psia) 68,9 KPa und bei einem inneren Rückfluß im Bereich von 0,2 bis 20 L/D:
(i) das Inkontaktbringen dieser Ströme in einer Destillationsreaktionszone mit einem Hydrierungskatalysator, hergestellt in einer derartigen Form, daß er als Destillationsstruktur wirkt, wodurch im wesentlichen sämtliche der besagten Diolefine und acetylenischen Verbindungen mit dem Wasserstoff unter Bildung von weniger ungesättigten Kohlenwasserstoffen in einer Reaktionsmischung reagieren, und
(ii) die Abtrennung der in dem ersten Strom enthaltenen Olefine und jeglicher Olefine, die durch besagte Hydrierung gebildet werden, aus der Reaktionsmischung durch fraktionierte Destillation.

12. Verfahren gemäß Anspruch 11, worin der erste Strom an dem unteren Ende der besagten Destillationsreaktionszone oder unterhalb desselben zugeführt wird.

13. Verfahren gemäß Anspruch 11, worin die besagten Ströme getrennt dem Destillationskolonnenreaktor zugeführt werden.

14. Verfahren gemäß Anspruch 11, worin besagte erste und zweite Ströme gemischt werden, bevor sie in den Destillationskolonnenreaktor eintreten.

15. Verfahren gemäß Anspruch 11, worin der Kohlenwasserstoffstrom aliphatische Verbindungen mit drei bis neun Kohlenstoffatomen umfaßt.

16. Verfahren gemäß Anspruch 15, worin der Kohlenwasserstoffstrom eine C₃-Fraktion umfaßt.

17. Verfahren gemäß Anspruch 15, worin der Kohlenwasserstoffstrom eine C₄-Fraktion umfaßt.

18. Verfahren gemäß Anspruch 15, worin der Kohlenwasserstoffstrom eine C₅-Fraktion umfaßt.

19. Verfahren gemäß Anspruch 15, worin der Kohlenwasserstoffstrom eine C₆-Fraktion umfaßt.

20. Verfahren gemäß Anspruch 15, worin die ersten und zweiten Ströme vor der Zufuhr zu dem Destillationskolonnenreaktor kombiniert werden.

21. Verfahren gemäß Anspruch 11, worin der Hydrierungskatalysator ein Metall der Gruppe VIII oder eine Verbindung des Metalls der Gruppe VIII als hauptsächliche katalytische Komponente umfaßt.

22. Verfahren gemäß Anspruch 21, worin der Hydrierungskatalysator 0,1 bis 5,0 Gew.% Palladiumoxid-auf-Aluminiumoxidextrudaten umfaßt.

23. Verfahren gemäß Anspruch 11, worin der Überkopfdruck des Destillationskolonnanreaktors zwischen (240 und 315 psig) 1754,93 und 2271,68 KPa liegt.

24. Verfahren gemäß Anspruch 23, worin der erste Strom Propylen umfaßt.

25. Verfahren gemäß Anspruch 11, worin die Destillationsstruktur ein Drahtgewebe aus flexiblem, halbstarrem, offenmaschigem, röhrenförmigem Material, gefüllt mit einem teilchenförmigen, hydrierungskatalytischen Material, umfaßt.

26. Verfahren gemäß Anspruch 11, das weiterhin als Stufe (c) das Abziehen der abgetrennten Olefine aus Stufe (b)(ii) zusammen mit jedweden Alkanen und leichteren Verbindungen einschließlich jedweden nichtumgesetzten Wasserstoffs aus dem Destillationskolonnenreaktor als Überkopfprodukt umfaßt.

27. Verfahren gemäß Anspruch 11, worin der Destillationskolonnenreaktor bei einem Überkopfdruck im Bereich zwischen (0 und 250 psig) 0 und 1823,83 KPa arbeitet.

28. Verfahren gemäß Anspruch 27, worin der Druck im Bereich von (50 bis 150 psig) 445,83 bis 1134,83 KPa liegt.

29. Verfahren gemäß Anspruch 11, worin die Temperatur innerhalb der Destillationsreaktionszone im Bereich von (40 bis 300°F) 4,44 bis 149°C liegt.

30. Verfahren gemäß Anspruch 29, worin die Temperatur innerhalb der Destillationsreaktionszone im Bereich von (110 bis 270°F) 43 bis 132°C liegt.

31. Verfahren gemäß Anspruch 11, worin im wesentlichen sämtliche der besagten Diolefine und acetylenischen Verbindungen aus dem Kohlenwasserstoffstrom eliminiert werden.

32. Verfahren gemäß Anspruch 11, worin der erste Strom eine WHSV im Bereich von 0,1 bis 35 besitzt.

33. Verfahren gemäß Anspruch 12, worin der Hydrierungskatalysator ein Metall der Gruppe VIII oder eine Verbindung eines Metalls der Gruppe VIII als hauptsächliche katalytische Komponente umfaßt.

34. Verfahren gemäß Anspruch 33, worin der Hydrierungskatalysator 0,1 bis 5,0 Gew.% Palladiumoxid-auf Aluminiumoxidextrudaten umfaßt.

35. Verfahren gemäß Anspruch 33, worin der Destillationskolonnenreaktor bei einem Überkopfdruck im Bereich zwischen (0 und 250 psig) 0 und 1823,83 KPa arbeitet.

36. Verfahren gemäß Anspruch 35, worin die Temperaturen innerhalb der Destillationsreaktionszone im Bereich von (40 bis 300°F) 4,44 bis 149°C liegen.

37. Verfahren gemäß Anspruch 36, worin der Druck im Bereich von (50 bis 150 psig) 445,83 bis 1134,83 KPa liegt.

38. Verfahren gemäß Anspruch 37, worin die Temperaturen innerhalb der Destillationsreaktionszone im Bereich von (110 bis 270°F) 43 bis 132°C liegen.

39. Verfahren gemäß Anspruch 38, worin der Wasserstoffpartialdruck im Bereich von (0,1 bis 7 psia) 0,69 bis 48,23 KPa liegt.

40. Verfahren gemäß Anspruch 39, worin der innere Rückfluß im Bereich von 0,5 bis 5 L/D liegt.

41. Verfahren gemäß Anspruch 11, worin im wesentlichen sämtliche der hoch ungesättigten Verbindungen hydriert werden.

42. Verfahren zur Isomerisierung von Olefinen, umfassend die Zufuhr eines Olefine enthaltenden Kohlenwasserstoffstroms zusammen mit einem Wasserstoffstrom bei einem Betriebswasserstoffpartialdruck im Bereich von zumindest (0,1 psia)0,69 KPa bis zu weniger als (10 psia) 68,9 KPa zu einem Destillationskolonnenreaktor, enthaltend einen Hydrierungskatalysator, welcher eine Komponente einer Destillationsstruktur ist, und die Isomerisierung eines Teils der isomerisierbaren Olefine sowie die Durchführung bei einem inneren Rückfluß im Bereich von 0,2 bis 20 L/D.

43. Verfahren gemäß Anspruch 42, worin besagter Kohlenwasserstoffstrom aliphatische Verbindungen mit vier bis neun Kohlenstoffatomen umfaßt.

44. Verfahren gemäß Anspruch 42, worin der Kohlenwasserstoff eine WHSV im Bereich von 0,1 bis 35 besitzt.

45. Verfahren gemäß Anspruch 43, worin der Überkopfdruck im Bereich von (0 bis 350 psig) 0 bis 2512,83 KPa liegt.

46. Verfahren gemäß Anspruch 42, worin der Wasserstoffpartialdruck geringer als (7 psia) 48,23 KPa ist.

47. Verfahren gemäß Anspruch 42, worin der Hydrierungskatalysator ein Metall der Gruppe VIII oder eine Verbindung eines Metalls der Gruppe VIII als hauptsächliche katalytische Komponente umfaßt.

48. Verfahren gemäß Anspruch 47, worin besagter Katalysator Pd umfaßt.

49. Verfahren gemäß Anspruch 45, worin der Überkopfdruck im Bereich von (50 bis 150 psia) 344,5 bis 1033,5 KPa liegt.

50. Verfahren gemäß Anspruch 42, worin der Kohlenwasserstoffstrom ein Leichtbenzin umfaßt.

51. Verfahren gemäß Anspruch 50, worin das Leichtbenzin 3-Methyl-buten-1 umfaßt.

52. Verfahren gemäß Anspruch 51, worin ein Teil von 3-Methyl-buten-1 zu 2-Methyl-buten-2 und 2-Methyl-buten-1 isomerisiert wird.

## Revendications

1. Un procédé pour l'hydrogénation sélective de composés très insaturés comprenant amener un courant d'hydrocarbures renfermant des monoléfines conjointement avec des composés très insaturés qui renferment des dioléfines et des acétylèniques avec un courant d'hydrogène sous une pression partielle d'hydrogène de travail dans la gamme d'au moins 0,69 Kpa (0,1 psia) à moins de 68,9 Kpa (10 psia) jusqu'à un réacteur d'une colonne de distillation renfermant un catalyseur d'hydrogénation qui est un composant d'une structure de distillation, à hydrogéner sélectivement une partie des composés très insaturés et à travailler avec un reflux interne dans la gamme de 0,2 à 20 L/D.

2. Le procédé selon la revendication 1, dans lequel ledit courant d'hydrocarbures comprend des composés aliphatiques ayant trois à neuf atomes de carbone.

3. Le procédé selon la revendication 1, dans lequel ledit courant d'hydrocarbures comprend une quantité majeure de n-oléfines qui sont récupérées comme des produits à partir de ladite hydrogénation.

4. Le procédé selon la revendication 1, dans lequel ledit hydrocarbure a une VSHP comprise dans la gamme de 0,1 à 35.

5. Le procédé selon la revendication 2, dans lequel la pression aérienne se situe dans la gamme de 0 à 2512,83 Kpa (0 à 350 psig).

6. Le procédé selon la revendication 1, dans lequel la pression partielle d'hydrogène est inférieure à 48,23 Kpa (7 psia).

7. Le procédé selon la revendication 5, dans lequel la pression partielle d'hydrogène est inférieure à 48,23 Kpa (7 psia).

8. Le procédé selon la revendication 1, dans lequel le catalyseur d'hydrogénation comprend un métal du Groupe VIII ou un composé de métal comme composant catalytique principal.

9. Le procédé selon la revendication 8, dans lequel ledit catalyseur comprend Pd.

10. Le procédé selon la revendication 5, dans lequel ladite pression aérienne se situe dans la gamme de 344,5 à 1033,5 Kpa (50 à 150 psia).

11. Un procédé pour l'hydrogénation sélective de composés très insaturés comprenant des monooléfines, des di-oléfines et des composés acétyléniques contenus dans un courant riche en oléfines, comprenant les étapes suivantes:
(a) amener (1) un premier courant comprenant des oléfines, des dioléfines et des composés hydrocarbonés acétyléniques et (2) un deuxième courant renfermant de l'hydrogène à un réacteur de colonne de distillation dans la zone d'alimentation ;
(b) concurremment dans ledit réacteur de colonne de distillation, sous une pression partielle d'hydrogène de 0,69 Kpa (0,1 psia) à moins de 68,9 Kpa (10 psia) et sous un reflux interne dans la gamme de 0,2 à 20 L/D :
(i) mettre en contact lesdits courants dans une zone de réaction de distillation avec un catalyseur d'hydrogénation préparée sous une forme pour agir comme une structure de distillation en faisant réagir ainsi essentiellement toutes lesdites dioléfines et les composés acétyléniques avec ledit hydrogène pour former moins d'hydrocarbures insaturés dans un mélange de réaction, et
(ii) séparer les oléfines contenues dans ledit premier courant et n'importe lesquelles des oléfines produites par ladite hydrogénation à partir dudit mélange de réaction par distillation fractionnée.

12. Le procédé selon la revendication 11, dans lequel ledit premier courant est amené au niveau ou en-dessous de l'extrémité inférieure de ladite zone de réaction de distillation.

13. Le procédé selon la revendication 11, dans lequel lesdits courants sont amenés séparément audit réacteur de colonne de distillation.

14. Le procédé selon la revendication 11, dans lequel lesdits premier et deuxième courants sont mélangés avant l'entrée dans ledit réacteur de colonne de distillation.

15. Le procédé selon la revendication 11, dans lequel ledit courant d'hydrocarbures comprend des composés aliphatiques ayant trois à neuf atomes de carbone.

16. Le procédé selon la revendication 15, dans lequel ledit courant d'hydrocarbures comprend une fraction en C₃.

17. Le procédé selon la revendication 15, dans lequel ledit courant d'hydrocarbures comprend une coupe en C₄.

18. Le procédé selon la revendication 15, dans lequel ledit courant d'hydrocarbures comprend une coupe en C₅.

19. Le procédé selon la revendication 15, dans lequel ledit courant d'hydrocarbures comprend une coupe en C₆.

20. Le procédé selon la revendication 15, dans lequel lesdits premier et deuxième courants sont combinés avant l'alimentation audit réacteur de colonne de distillation.

21. Le procédé selon la revendication 11, dans lequel le catalyseur d'hydrogénation comprend un métal du Groupe VIII ou un composé de métal comme le principal composant catalytique.

22. Le procédé selon la revendication 21, dans lequel ledit catalyseur d'hydrogénation comprend de 0,1 à 5 % en poids d'oxyde de palladium fixé sur des extrudats d'alumine.

23. Le procédé selon la revendication 11, dans lequel la pression aérienne dudit réacteur de colonne de distillation se situe entre 1754,93 et 2271,68 Kpa (240 et 315 psig).

24. Le procédé selon la revendication 23, dans lequel ledit premier courant comprend du propylène.

25. Le procédé selon la revendication 11, dans lequel ladite structure de distillation comprend un treillis de matériau tubulaire souple à mailles ouvertes semi-rigide, garni d'un matériau catalytique d'hydrogénation particulaire.

26. Le procédé selon la revendication 11 comprenant, en outre, l'étape (c) consistant à retirer les oléfines séparées de l'étape (b) (ii) avec n'importe quels alcanes et composés plus légers, y compris n'importe quel hydrogène n'ayant pas réagi, à partir dudit réacteur de colonne de distillation, comme produits aériens.

27. Le procédé selon la revendication 11, dans lequel ledit réacteur de colonne de distillation agit à une pression aérienne comprise dans la gamme de 0 à 1823,83 Kpa (0 et 250 psig).

28. Le procédé selon la revendication 27, dans lequel ladite pression se situe dans la gamme de 445,83 à 1134,83 Kpa (50 à 150 psig).

29. Le procédé selon la revendication 11, dans lequel les températures dans ladite zone de réaction de distillation se situent dans la gamme de 4,44 à 149° C (40 à 300° F).

30. Le procédé selon la revendication 29, dans lequel les températures dans ladite zone de réaction de distillation se situent dans la gamme de 43 à 132° C (110 à 270° F).

31. Le procédé selon la revendication 11, dans lequel pratiquement tous lesdits composés acétyléniques et lesdites dioléfines sont éliminés dudit courant d'hydrocarbures.

32. Le procédé selon la revendication 11, dans lequel le premier courant a une VSHP comprise dans la gamme de 0,1 à 35.

33. Le procédé selon la revendication 12, dans lequel le catalyseur d'hydrogénation comprend un métal du Groupe VIII ou un composé de métal comme composant catalytique principal.

34. Le procédé selon la revendication 33, dans lequel ledit catalyseur d'hydrogénation comprend de 0,1 à 5,0 % en poids d'oxyde de palladium fixé sur des extrudats d'alumine.

35. Le procédé selon la revendication 33, dans lequel ledit réacteur de colonne de distillation agit à une pression aérienne comprise dans la gamme de 0 à 1823,83 Kpa (0 et 250 psig).

36. Le procédé selon la revendication 35, dans lequel les températures dans ladite zone de réaction de distillation se situent dans la gamme de 4,44 à 149° C (40 à 300° F).

37. Le procédé selon la revendication 36, dans lequel ladite pression se situe dans la gamme de 445,83 à 1134,83 Kpa (50 à 150 psig).

38. Le procédé selon la revendication 37, dans lequel les températures dans ladite zone de réaction de distillation se situent dans la gamme de 43 à 132° C (110 à 270° F).

39. Le procédé selon la revendication 38, dans lequel la pression partielle d'hydrogène se situe dans la gamme de 0,69 à 48,23 Kpa (0,1 à 7 psia).

40. Le procédé selon la revendication 39, dans lequel il y a un reflux interne dans la gamme de 0,5 à 5 L/D.

41. Le procédé selon la revendication 11, dans lequel pratiquement tous les composés très insaturés sont hydrogénés.

42. Procédé d'isomérisation d'oléfines comprenant l'alimentation d'un courant d'hydrocarbures renfermant des oléfines avec un courant d'hydrogène à une pression partielle d'hydrogène de travail dans la gamme d'au moins 0,69 Kpa (0,1 psia) à moins de 68,9 Kpa (10 psia) jusqu'à un réacteur de colonne de distillation renfermant un catalyseur d'hydrogénation qui est un composant d'une structure de distillation et l'isomérisation d'une partie des oléfines aptes à être isomérisées et la mise en oeuvre d'un reflux interne dans la gamme de 0,2 à 20 L/D.

43. Le procédé selon la revendication 42, dans lequel ledit courant d'hydrocarbures comprend des composés aliphatiques ayant quatre à neuf atomes de carbone.

44. Le procédé selon la revendication 42, dans lequel ledit hydrocarbure a une VSHP comprise dans la gamme de 0,1 à 35.

45. Le procédé selon la revendication 43, dans lequel la pression aérienne se situe dans la gamme de 0 à 2512,83 Kpa (0 à 350 psig).

46. Le procédé selon la revendication 42, dans lequel la pression partielle d'hydrogène est inférieure à 48,23 Kpa (7 psia).

47. Le procédé selon la revendication 42, dans lequel le catalyseur d'hydrogénation comprend un métal du Groupe VIII ou un composé de métal comme composant catalytique principal.

48. Le procédé selon la revendication 47, dans lequel ledit catalyseur comprend Pd.

49. Le procédé selon la revendication 45, dans lequel ladite pression aérienne se situe dans la gamme de 344,5 à 1033,5 Kpa (50 à 150 psia).

50. Le procédé selon la revendication 42, dans lequel ledit courant d'hydrocarbures comprend un naphta léger.

51. Le procédé selon la revendication 50, dans lequel ledit naphta léger comprend le 3-méthyl butène-1.

52. Le procédé selon la revendication 51, dans lequel une partie du 3-méthyl butène-1 est isomérisée en 2-méthyl butène 2 et 2-méthyl butène-1.
